# EUROPÄISCHE PATENTANMELDUNG

(11) **EP 1 378 851 A1**
(43) Veröffentlichungstag der Anmeldung: **07.01.2004**
(21) Anmeldenummer: 02014703.9
(22) Anmeldetag: 03.07.2002
(51) Int. Cl.: G06F 19/00

(54) **Verfahren und System zur Unterstützung bei der Auswahl eines Trainingsprogramms im Rahmen einer Therapieplanung**

(71) Anmelder: SIEMENS AKTIENGESELLSCHAFT, 80333 München (DE); Dr. Hein GmbH, 90429 Nürnberg (DE)
(72) Erfinder: Abraham-Fuchs, Klaus, 91058 Erlangen (DE); Eisermann, Uwe, 91052 Erlangen (DE); Hein, Achim, 90403 Nürnberg (DE); Richter, Nils, 95349 Thurnau (DE); Setz, Robert, 91126 Rednitzhembach (DE)
(74) Vertreter: Berg, Peter, Dipl.-Ing.

(57) **Zusammenfassung**

Die vorliegende Erfindung betrifft ein Verfahren sowie ein System zur Unterstützung bei der Auswahl eines Trainingsprogramms im Rahmen einer Therapieplanung. Bei dem Verfahren wird ein Fähigkeitsprofil eines Patienten sowie eine erste Datenbank (11) bereitgestellt, die mehrere zurückliegende Vergleichs-Fähigkeitsprofile von Vergleichspatienten und eine Zuordnung von jeweils auf Basis dieser Vergleichs-Fähigkeitsprofile verordneten Trainingsprogrammen enthält. Von einer Datenverarbeitungsstation (10) wird das Fähigkeitsprofil des Patienten automatisch mit den Vergleichs-Fähigkeitsprofilen verglichen und ein oder mehrere geeignete Trainingsprogramme aus der ersten Datenbank (11) ausgewählt und angezeigt, bei denen das zugeordnete Vergleichs-Fähigkeitsprofil mit dem Fähigkeitsprofil des Patienten in einem vorgebbaren Ähnlichkeitsmaß übereinstimmt. Das Verfahren sowie das zugehörige System erleichtern dem Arzt oder Therapeuten die Auswahl eines individuellen Trainingsprogramms.

## Beschreibung

Die vorliegende Erfindung betrifft ein Verfahren sowie ein System zur Unterstützung bei der Auswahl eines Trainingsprogramms im Rahmen einer Therapieplanung für die Rehabilitation eines Patienten.

Als Folge schwerer Erkrankungen wie bspw. Schlaganfall, Herzinfarkt oder Alzheimer sche Krankheit oder als Folge schwerer operativer Eingriffe wie bspw. dem Einsatz von Gelenkimplantaten oder der Durchführung einer Amputation treten bei der Mehrzahl der Patienten unterschiedliche Defizite in der körperlichen und geistigen Leistungsfähigkeit auf. Diese Defizite sind in der Regel die Folge der Schwächung oder des kompletten Ausfalls einer Gehirnregion oder eines Muskels. Auch Kombinationen hiervon treten häufig auf. So kann z. B. eine Gehirnregion geschädigt sein, die für die Steuerung eines Muskels oder mehrerer Muskeln in funktionalen Ketten zuständig ist. Als Folge davon degenerieren die betroffenen Muskeln, so dass sie nicht mehr richtig eingesetzt werden können. Solche geistigen oder körperlichen Einschränkungen werden in der medizinischen Fachsprache als Fähigkeitsdefizite bezeichnet, die in verschiedene Fähigkeitsbereiche eingeteilt werden können. So ist unterscheidet eine bekannte Klassifizierung bspw.:
- motorische Fähigkeiten wie Kraft, Ausdauer, Beweglichkeit, Gleichgewicht, Reaktion, Orientierung, Differenzierung, Umstellung, Sprachmotorik;
- intellektuelle/kognitive Fähigkeiten wie Aufmerksamkeit, Gedächtnis, Planung, Sprachverständnis, Wortfindung, Sehen;
- organisch/physische Fähigkeiten wie bspw. die Verringerung der Organleistung;
- soziale Fähigkeiten wie bspw. die Kommunikations- und Partizipationsfähigkeit;
- emotionale Fähigkeiten wie bspw. die Fähigkeit zur Entwicklung des Selbstwertgefühls.
Einige Fähigkeiten erfordern auch ein Zusammenspiel motorischer und kognitiver Funktionen. So setzt bspw. die Tätigkeit des Treppensteigens Kraft und Gleichgewicht als motorische Fähigkeiten sowie Aufmerksamkeit und räumliche Wahrnehmung als kognitive Fähigkeiten voraus.

Sehr häufig tritt bei einem Patienten nicht ein einziges Defizit in einer Fähigkeitskategorie auf, sondern eine Kombination mehrerer Defizite in mehr oder weniger schwerer Ausprägung. Ziel einer therapeutischen Maßnahme, die meist im Rahmen eines Rehabilitationsprozesses durchgeführt wird, ist die Wiederherstellung der Fähigkeiten bzw. die weitest mögliche Verminderung der vorliegenden Defizite. Zu Beginn der rehabilitativen Maßnahme werden dabei in der Regel alle Fähigkeitsdefizite des Patienten anhand bekannter Verfahren zur Messung, Beobachtung und Befragung erfasst und ihr Ausmaß dokumentiert. Dieser Erfassungsprozess wird auch als Staging des Patienten bezeichnet. Je nach eingesetztem Messverfahren ist das Ergebnis dieses Staging-Prozesses quantitativ, bspw. in Prozent der Sehfähigkeit oder durch Angabe des Grades der Beweglichkeit des Oberarmes, oder qualitativ, bspw. durch eine Einstufung der Fähigkeitseinschränkung in schwer, mittelgradig oder leicht. Ein Beispiel eines etablierten Messverfahrens für das Staging zahlreicher neurologischer, kognitiver und psychischer Fähigkeiten ist die sog. Wiener Testbatterie der Firma Schuhfried.

Das Resultat dieser Eingangsuntersuchung ist im Idealfall ein fachübergreifender Fähigkeitsbericht, der sich in Form eines Fähigkeitsprofils darstellen lässt. Ein Fähigkeitsprofil wird in diesem Kontext als eine Liste aller relevanten Fähigkeiten und eine Zuordnung des Grades der Einschränkung dieser Fähigkeiten bei diesem Patienten zum Erhebungszeitpunkt definiert.

Neben dem Begriff Fähigkeiten wird in der medizinischen Fachsprache auch der Begriff Fertigkeit benutzt. Unter einer Fertigkeit wird im Kontext einer medizinischen Rehabilitationsmaßnahme eine komplexe Handlung verstanden, die aber in sich geschlossen und gegenüber anderen Handlungen abgrenzbar ist. Für eine Fertigkeit wird das Zusammenspiel mehrerer Fähigkeiten benötigt. Insbesondere bezieht sich der Begriff Fertigkeit im Kontext einer Rehabilitation auf Aktivitäten des täglichen Lebens (ADL: Activities of Daily Living), die Grundvoraussetzung für ein unabhängiges, selbständiges Leben sind. Beispiele für derartige Fertigkeiten sind das Einnehmen von Nahrung, das Anziehen, das Waschen, das Duschen, das Treppensteigen usw.. Die Ausführung solcher Fertigkeiten wird auch in standardisierten Fragebögen erfasst und als ADL-Index quantifiziert. Obwohl im Rahmen einer Rehabilitation in direkter Weise Fähigkeiten trainiert werden, ist das eigentliche Ziel die Wiedererlangung von Fertigkeiten. Insofern sind im Kontext der nachfolgenden Beschreibung die Begriffe Fähigkeit und Fertigkeit meist gegenseitig austauschbar.

In der Rehabilitation von krankheits- oder unfallbedingten Fertigkeits- oder Fähigkeitsdefiziten muss für den Patienten ein individuelles Trainingsprogramm auf Basis der vorliegenden Defizite zusammengestellt werden. Ein Trainingsprogramm kann dabei die Durchführung einer einzelnen Übung mit einer individuell gewählten Steigerung des Schwierigkeitsgrades im Laufe der Zeit oder auch die Durchführung mehrerer Übungen mit bestimmter Gewichtung und in einer bestimmten Reihenfolge umfassen. Das individuelle Trainingsprogramm wird vom Arzt oder Therapeuten aus seiner Erfahrung heraus ausgewählt bzw. zusammengestellt. Diese Erfahrung beruht zum einen auf Lehrbuchwissen, zum anderen auf der Beobachtung vieler Patienten im Laufe seiner Berufsausübung. Der Arzt knüpft hierbei in der Regel intuitiv an die Erfahrungen mit einem möglichst ähnlich gelagerten Fall an und überträgt ein dort erfolgreiches Trainingsprogramm auf den neuen Fall. Hierbei ist der Arzt jedoch auf sein Gedächtnis und ggf. vorhandene Aufzeichnungen angewiesen. Andererseits kann der Arzt nur dann auf bisherige Erfahrungen zurückgreifen, wenn er bereits ausreichend Patienten mit vergleichbaren Fähigkeitsdefiziten behandelt hat.

Die Aufgabe der vorliegenden Erfindung besteht darin, ein Verfahren sowie ein System zur Unterstützung bei der Auswahl eines Trainingsprogramms in der Therapieplanung anzugeben, das die Arbeit für den Arzt oder Therapeuten erleichtert und unabhängig von seinen individuellen bisherigen Erfahrungen in der Verordnung von Trainingsprogrammen macht.

Die Aufgabe wird mit dem Verfahren sowie dem System der Patentansprüche 1 bzw. 13 gelöst. Vorteilhafte Ausgestaltungen des Verfahrens sowie des Systems sind Gegenstand der Unteransprüche oder lassen sich der nachfolgenden Beschreibung sowie den Ausführungsbeispielen entnehmen.

Bei dem vorliegenden Verfahren zur Unterstützung bei der Auswahl eines Trainingsprogramms in der Therapieplanung werden ein Fähigkeitsprofil eines Patienten sowie eine erste Datenbank bereitgestellt, die mehrere zurückliegende Vergleichs-Fähigkeitsprofile von Vergleichspatienten und eine Zuordnung von jeweils auf Basis dieser Vergleichs-Fähigkeitsprofile verordneten Trainingsprogrammen enthält. Eine Datenverarbeitungsstation vergleicht automatisch das Fähigkeitsprofil des Patienten mit den Vergleichs-Fähigkeitsprofilen, wählt auf Basis dieses Vergleichs mehrere geeignete Trainingsprogramme aus der ersten Datenbank aus, bei denen das zugeordnete Vergleichs-Fähigkeitsprofil mit dem Fähigkeitsprofil des Patienten in einem vorgebbaren Ähnlichkeitsmaß übereinstimmt, und zeigt diese an.

Die erste Datenbank enthält dabei die Vergleichs-Fähigkeitsprofile sowie jeweils verordneten Trainingsprogramme eines wesentlich größeren Patientenkollektivs als dies aus der Tätigkeit eines einzelnen Arztes resultieren kann. Der persönliche Erfahrungsbereich des behandelnden Arztes oder Therapeuten, der in die Entscheidung über die Zusammenstellung eines Trainingsprogramms eingeht, wird durch das vorliegende Verfahren erheblich ausgeweitet, indem die Erfahrungen von weiteren Ärzten und einem sehr viel größeren Patientenkollektiv in die Entscheidung einbezogen werden. Damit erfolgt die Auswahl des Trainingsprogramms beim vorliegenden Verfahren und zugehörigen System nicht mehr intuitiv erfahrungsbasiert sondern evidenzbasiert. Insbesondere ist die Qualität der Trainingsverschreibung nicht mehr in gleichem Maße abhängig von der Erfahrung des jeweiligen Arztes wie dies bisher der Fall ist.

Das vorgebbare Ähnlichkeitsmaß, mit dem das Vergleichs-Fähigkeitsprofil mit dem Fähigkeitsprofil des Patienten übereinstimmen muss kann entweder vom Arzt oder Therapeuten vorgegeben werden oder durch die Datenverarbeitungsstation festgelegt sein oder festgelegt werden. Das Ähnlichkeitsmaß kann hierbei bspw. so festgelegt sein, dass die Vergleichs-Fähigkeitsprofile in den gleichen Fähigkeiten Defizite aufweisen müssen als das Fähigkeitsprofil eines Patienten. Weiterhin kann auch eine Maximalanzahl vom Fähigkeitsprofil des Patienten abweichender Fähigkeitsdefizite als vorgebbares Ähnlichkeitsmaß der Übereinstimmung angegeben werden. Schließlich lässt sich auch der Grad der einzelnen Defizite durch Angabe von Bereichen einschränken, in denen jeweils die Vergleichs-Fähigkeitsprofile mit dem Fähigkeits-Profil des Patienten übereinstimmen müssen, damit von der Datenverarbeitungsstation das jeweils zugeordnete Trainingsprogramm ausgewählt und angezeigt wird. Das Ähnlichkeitsmaß läßt sich dabei z. B. mathematisch als Vektor in einem durch oben genannte Kriterien aufgespannten Raum darstellen, wobei dann der Winkel zwischen den Vektoren sowie die Länge der Vektoren verglichen werden können.

In einer weiteren Ausbildung des vorliegenden Verfahrens sowie des zugehörigen Systems sind in der ersten Datenbank zusätzlich Maßzahlen für den Behandlungserfolg bei den jeweiligen Vergleichspatienten enthalten, die dem jeweiligen, dem Patienten verordneten Trainingsprogramm zugeordnet sind. Diese Maßzahlen für den Behandlungserfolg des jeweils verordneten Trainingsprogramms werden vorzugsweise durch die Datenverarbeitungsstation zusammen mit dem bzw. den ausgewählten geeigneten Trainingsprogrammen angezeigt.

Diese Anzeige kann in einer Ausführungsform des vorliegenden Verfahrens in der Reihenfolge der Größe der zugeordneten Maßzahlen für den Behandlungserfolg erfolgen, so dass der Arzt oder Therapeut an seinem Computerarbeitsplatz an oberster Stelle das Trainingsprogramm vorfindet, das in der Vergangenheit bei einem vergleichbaren Patienten das beste Behandlungsergebnis erzielt hat. In einer weiteren Ausgestaltung des vorliegenden Verfahrens wird die Reihenfolge der Anzeige der ausgewählten Trainingsprogramme durch die Datenverarbeitungsstation entsprechend dem Grad der Übereinstimmung der Vergleichs-Fähigkeitsprofile mit dem Fähigkeitsprofil des Patienten gewählt. Weiterhin ist es auch möglich, durch die Datenverarbeitungsstation nur die Trainingsprogramme anzeigen zu lassen, die die höchste oder eine oberhalb eines vorgebbaren Schwellwertes liegende Maßzahl für den Behandlungserfolg aufweisen. In gleicher Weise können das Verfahren und das System so ausgebildet sein, dass automatisch ein Vorschlagfür ein Trainingsprogramm ausgegeben wird, das auf Basis der Übereinstimmung der Vergleichs-Fähigkeitsprofile mit dem Fähigkeitsprofil des Patienten sowie der zugeordneten Maßzahlen für den Behandlungserfolg nach vorgebbaren Auswahlregeln von der Datenverarbeitungsstation ausgewählt wird, die vorzugsweise von Experten erstellt werden. Die Auswahlregeln beinhalten beispielsweise "wenn-dann"-Entscheidungen in Verbindung mit Schwellwerten für das Ähnlichkeitsmaß der Übereinstimmung der Vergleichs-Fähigeitsprofile mit dem Fähigkeitsprofil des Patienten und für die Maßzahlen für den Behandlungserfolg. Ein Trainingsprogramm ist hierbei charakterisiert durch den Namen von Trainingsmodulen, die Dauer der Ausübung und die zeitliche Abfolge der Trainingsmodule sowie weitere Parameter wie z. B. den Schwierigkeitsgrad, die die einzelnen Module genauer spezifizieren.

In einer weiteren Ausgestaltung des vorliegenden Verfahrens sowie des zugehörigen Systems wird durch die Datenverarbeitungsstation neben dem Vergleich der Fähigkeits-Profile automatisch eine Übereinstimmung weiterer Patientendaten des Patienten mit Patientendaten der Vergleichs-Patienten geprüft und bei der Auswahl berücksichtigt. Diese weiteren Patientendaten müssen allerdings zusätzlich in der ersten Datenbank enthalten sein. Weitere Patientendaten, die für die Auswahl eines Trainingsprogramms relevant sein können, sind bspw. das Alter und Geschlecht des Patienten, sein Bildungsstand, seine Berufsgruppe sowie andere körperliche oder geistige Einschränkungen wie bspw. Diabetes, ein Herzschrittmacher o. ä.. Die weiteren Patientendaten des Patienten selbst können dabei entweder von der Datenverarbeitungsstation an einer Eingabestation abgefragt werden, so dass sie durch den Arzt oder Therapeuten eingegeben werden müssen. Sie können auch aus einer elektronischen Patientenakte automatisch abgerufen werden, auf die die Datenverarbeitungsstation Zugriff hat. Die Datenverarbeitungsstation wählt in diesem Fall nur die Trainingsprogramme aus, die zu Vergleichspatienten gehören, bei dem diese Randbedingungen, d. h. die weiteren Patientendaten neben dem Fähigkeitsprofil mit den entsprechenden Daten des Patienten übereinstimmen.

Da das Fähigkeitsprofil sowie ggf. weitere individuelle Randbedingungen in den Patientendaten des Patienten sehr unterschiedlich sein können, kann der ähnlichste aufgefundene Vergleichsfall evtl. immer noch signifikante Unterschiede zum aktuellen Fall aufweisen, so dass das zugehörige Trainingsprogramm nicht direkt übernommen werden kann. Der Arzt oder Therapeut hat hier die Möglichkeit, die vorgeschlagenen oder angezeigten geeigneten Trainingsprogramme zusätzlich zu modifizieren, um sie an den aktuellen Fall anzupassen. Die Modifikation eines derartigen Trainingsvorschlages bringt in jedem Fall einen geringeren Arbeitsaufwand als eine komplette neue Zusammenstellung eines Trainingsprogramms.

Die Nutzung der Datenbank mit den Vergleichspatienten hat den besonderen Vorteil, dass sie mit jedem neu behandelten Patienten ständig anwachsen kann. Somit wird einerseits die Wahrscheinlichkeit ständig größer, dass ein früherer Fall mit einem sehr ähnlichen Vergleichs-Fähigkeitsprofil gefunden wird. Andererseits wird immer automatisch das Trainingsprogramm vorgeschlagen, das in der Vergangenheit das beste Ergebnis, den besten Outcome, erzeugt hat. Das vorgeschlagene System ist also in diesem Sinne selbstlernend.

Die Datenbank mit den historischen Patientendaten der Vergleichs-Patienten kann selbstverständlich anonymisiert sein, da der Bezug zum Namen des jeweiligen Vergleichspatienten bei der weiteren Verwendung dieser Daten im Rahmen des vorliegenden Verfahrens nicht erforderlich ist. Alle Fähigkeitsprofile werden beim vorliegenden Verfahren in gleicher Art und Weise gemäß existierender und anerkannter Bewertungsschemata, bspw. gemäß dem Staffelstein-Index, dem Bartel-Index, usw., erhoben. Die Maßzahl für den Behandlungserfolg, der sog. Outcome, wird ebenfalls gemäß einem standardisierten Verfahren bei allen Patienten in gleicher Art und Weise gemessen, um vergleichbare Werte zu erhalten. Geeignete Maßzahlen für den Behandlungserfolg können über das Fähigkeitsprofil, das Fertigkeitsprofil, den Lebensqualitäts-Index, die Ergebnisses von Staging-Tests, die Ergebnisse der Trainingsübungen usw. erhalten werden, wobei vorzugsweise die Differenz der Maßzahlen zum jeweiligen Ausgangszustand vor Beginn des entsprechenden Trainingsprogramms gemessen wird. Selbstverständlich kann die Maßzahl für den Outcome auch aus einer gewichteten Kombination bzw. Mittelung mehrerer Teilmaßzahlen gebildet sein.

In einer weiteren vorteilhaften Ausführungsform des vorliegenden Verfahrens werden wiederholt im Verlauf einer Therapie ein aktuelles Fähigkeitsprofil des Patienten bereitgestellt und von der Datenverarbeitungsstation automatisch die vorangehend beschriebenen Verfahrensschritte durchgeführt. Durch diese erneute Suche nach dem nunmehr ähnlichsten Fähigkeitsprofil in der ersten Datenbank kann sich eine Abweichung von dem bisher verordneten Trainingsprogramm ergeben. In diesem Falle erfolgt ein Vorschlag für ein neues Trainingsprogramm, das unter den inzwischen veränderten Randbedingungen evidenzbasiert das in der Vergangenheit, d. h. auf Basis der Daten der ersten Datenbank, das Beste war. Dieser Vorschlag für ein neues Trainingsprogramm kann als Ausgangsbasis für die Modifikation des bestehenden Trainingsprogramms dienen. Selbstverständlich ist als weitere Randbedingung darauf zu achten, dass eine gleitende Überführung des alten Trainingsprogramms in das neue Trainingsprogramm ohne Diskontinuitätsprünge gewährleistet ist. Dies kann durch einen entsprechenden automatisierten Vorschlag der Datenverarbeitungsstation durch Rückgriff auf eine dritte Datenbank erfolgen, die entsprechende Überführungsmaßnahmen zwischen unterschiedlichen Trainingsprogrammen enthält.

Das vorliegende System zur Unterstützung der Therapieplanung in der Auswahl eines Trainingsprogramms umfasst entsprechend eine Datenverarbeitungsstation, die mit der ersten Datenbank verbunden ist und ein Modul zum automatischen Vergleich des-Fähigkeitsprofils des Patienten mit den Vergleichs-Fähigkeitsprofilen und zur Auswahl und Anzeige eines oder mehrerer geeigneter Trainingsprogramme aus der ersten Datenbank, bei denen das zugeordnete Vergleichs-Fähigkeitsprofil mit dem Fähigkeitsprofil des Patienten in einem vorgebbaren Ähnlichkeitsmaß übereinstimmt.

In den weiteren Ausgestaltungen des vorliegenden Systems ist die Datenverarbeitungsstation mit einer oder mehreren der weiteren Datenbanken verbunden und der Inhalt der ersten Datenbank sowie das Modul sind entsprechend zur Durchführung der vorangehend erläuterten Verfahrensschritte ausgebildet.

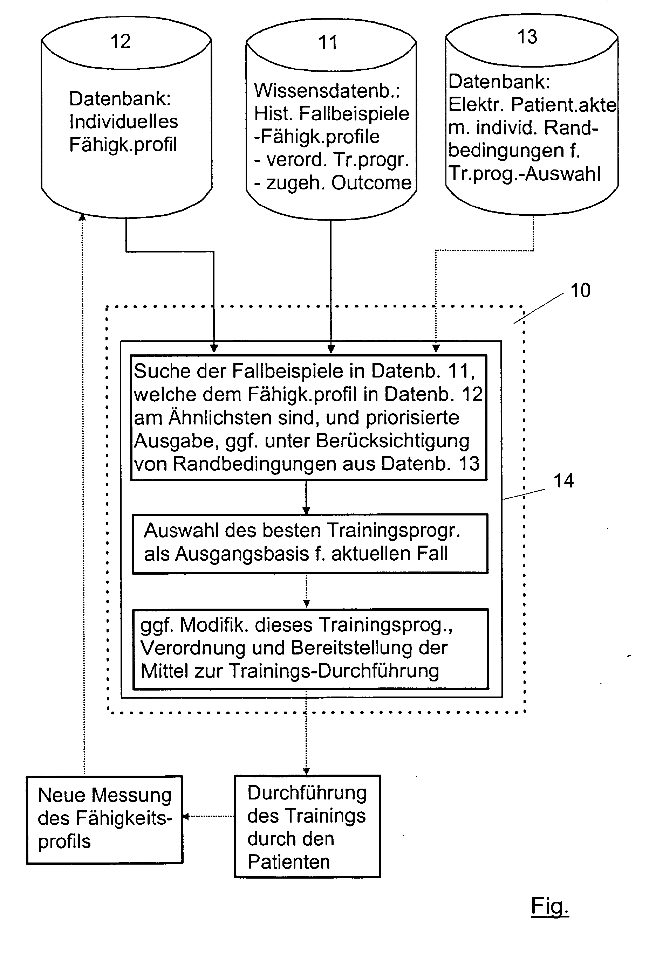

Das vorliegende Verfahren und das zugehörige System werden nachfolgend anhand eines Ausführungsbeispiels in Verbindung mit der Zeichnung ohne Beschränkung des allgemeinen Erfindungsgedankens nochmals erläutert.

Die Figur zeigt einen Überblick über das vorliegende Verfahren sowie das zugehörige System in einer Ausgestaltung der Erfindung. Im vorliegenden Ausführungsbeispiel wird der Vorgang der Erstellung eines Trainingsprogramms in der Therapieplanung durch einen Arzt oder Therapeuten unter Einsatz des vorliegenden Verfahrens sowie des zugehörigen Systems in einer speziellen Ausgestaltung beispielhaft verdeutlicht. Das beispielhafte System umfasst einen Computerarbeitsplatz (Datenverarbeitungsstation 10) zur Therapieplanung mit einem Modul 14 zur automatisierten Verarbeitung der durch die Datenbanken zur Verfügung gestellten Daten. Die Datenverarbeitungsstation 10 ist hierbei mit einer ersten Wissens-Datenbank 11 verbunden, die historische Fallbeispiele mit Vergleichs-Patienten und deren Vergleichs-Fähigkeitsprofilen, den auf Basis dieser Vergleichs-Fähigkeitsprofile verordneten Trainingsprogrammen sowie den mit dem jeweiligen Trainingsprogramm erzielten Behandlungserfolg, den Outcome, enthält. Das Maß für den Behandlungserfolg, der Outcome, wird hierbei vorzugsweise als Verbesserung der Fähigkeitsdefizite pro Zeiteinheit mit einer entsprechenden Maßzahl angegeben. Die Datenverarbeitungsstation 10 ist weiterhin mit einer zweiten Datenbank 12 verbunden, die das individuelle Fähigkeitsprofil des zu behandelnden Patienten enthält. Optional kann die Datenverarbeitungsstation 10 noch mit einer dritten Datenbank 13 verbunden sein, die bspw. in Form einer elektronischen Patientenakte, individuelle Randbedingungen des zu behandelnden Patienten für eine Trainingsprogrammauswahl enthält, wie bspw. vorliegende Krankheiten oder sonstige körperliche oder geistige Einschränkungen, das Alter, das Gewicht oder das Geschlecht.

Bei der Durchführung des vorliegenden Verfahrens werden bei diesem Ausführungsbeispiel mit Hilfe eines geeignet gewählten mathematischen Abstandsmaßes durch das Modul 14 aus der ersten Wissens-Datenbank 11 diejenigen Fälle ausgewählt, die dem aktuellen Fall am ähnlichsten sind. Die Auswahl erfolgt vorzugsweise in der Reihenfolge der Ähnlichkeit. Aus diesen ausgewählten ähnlichsten Fällen werden wiederum diejenigen priorisiert, die den besten Outcome aufweisen. Die solcher Maßen priorisierten Vorschläge werden schließlich an einem Monitor angezeigt, so dass sie den das Verfahren einsetzenden Arzt oder Therapeuten als beste Alternativen für eine evidenzbasierte Auswahl eines Trainingsprogramms für den aktuellen Patienten zur Entscheidungsunterstützung vorliegen. Der Arzt oder Therapeut verordnet dann das entsprechende ggf. zusätzlich durch ihn modifizierte Trainingsprogramm. Dem Patienten werden dann entsprechend der Verordnung die Mittel zur Trainingsdurchführung bereitgestellt.

Während der Durchführung des Trainingsprogramms durch den Patienten wird wiederholt ein Fähigkeitsprofil des Patienten erfasst und in der zweiten Datenbank 12 abgespeichert. Diese Erfassung des Fähigkeitsprofils kann in regelmäßigen Zeitabständen oder nach vorgebbaren Zeiten erfolgen. Das Modul 14 der Datenverarbeitungsstation 10 ruft dann jeweils das aktuelle Fähigkeitsprofil des Patienten von der zweiten Datenbank 12 ab und führt den Vergleich mit den historischen Patientendaten erneut durch. Auf Basis dieses Vergleichs wird dann ggf. eine Modifikation des verordneten Trainingsprogramms vorgeschlagen, die der Arzt oder Therapeut wiederum übernehmen, modifizieren oder ablehnen kann.

Durch das vorliegende Verfahren und das zugehörige System wird der Arzt oder Therapeut in der individuellen Erstellung eines Trainingsprogramms für einen Patienten unterstützt. Die ihm hierbei vorgeschlagene Auswahl an Trainingsprogrammen erfolgt evidenzbasiert und automatisiert. Durch die gleichzeitige Information über die mit den vorgeschlagenen Trainingsprogrammen erzielten Behandlungserfolge lässt sich jederzeit das aus historischer Sicht für den Patienten geeignetste Trainingsprogramm auswählen.

## Patentansprüche

1. Verfahren zur Unterstützung bei der Auswahl eines Trainingsprogramms im Rahmen einer Therapieplanung, bei dem ein Fähigkeitsprofil eines Patienten und eine erste Datenbank (11) bereitgestellt werden, die mehrere zurückliegende Vergleichs-Fähigkeitsprofile von Vergleichspatienten und eine Zuordnung von jeweils auf Basis dieser Vergleichs-Fähigkeitsprofile verordneten Trainingsprogrammen enthält, und von einer Datenverarbeitungsstation (10) automatisch das Fähigkeitsprofil des Patienten mit den Vergleichs-Fähigkeitsprofilen verglichen und ein oder mehrere geeignete Trainingsprogramme aus der ersten Datenbank (11) ausgewählt und angezeigt werden, bei denen das zugeordnete Vergleichs-Fähigkeitsprofil mit dem Fähigkeitsprofil des Patienten in einem vorgebbaren Ähnlichkeitsmaß übereinstimmt.

2. Verfahren nach Anspruch 1,
**dadurch gekennzeichnet,**
**dass** den verordneten Trainingsprogrammen in der ersten Datenbank (11) zusätzlich Maßzahlen für den Behandlungserfolg beim jeweiligen Vergleichspatienten zugeordnet sind und die ein oder mehreren geeigneten Trainingsprogramme zusammen mit der zugeordneten, gegebenenfalls gemittelten Maßzahl für den Behandlungserfolg angezeigt werden.

3. Verfahren nach Anspruch 2,
**dadurch gekennzeichnet,**
**dass** die geeigneten Trainingsprogramme in der Reihenfolge der Größe der zugeordneten Maßzahlen für den Behandlungserfolg dargestellt werden.

4. Verfahren nach Anspruch 1 oder 2,
**dadurch gekennzeichnet,**
**dass** die geeigneten Trainingsprogramme in der Reihenfolge des Ähnlichkeitsmaßes der Übereinstimmung der zugeordneten Vergleichs-Fähigkeitsprofile mit dem Fähigkeitsprofil des Patienten dargestellt werden.

5. Verfahren nach einem der Ansprüche 2 bis 4,
**dadurch gekennzeichnet,**
**dass** nur das oder die geeigneten Trainingsprogramme mit der höchsten Maßzahl für den Behandlungserfolg oder mit einer über einem vorgebbaren Schwellwert liegenden Maßzahl für den Behandlungserfolg dargestellt werden.

6. Verfahren nach einem der Ansprüche 1 bis 5,
**dadurch gekennzeichnet,**
**dass** die Datenverarbeitungsstation auf Basis der Übereinstimmung der Vergleichs-Fähigkeitsprofile mit dem Fähigkeitsprofil des Patienten und der Maßzahlen für den Behandlungserfolg der jeweils verordneten Trainingsprogramme nach vorgebbaren Auswahlregeln ein geeignetes Trainingsprogramm vorschlägt, das ein optimales Trainingsergebnis bei dem Patienten erwarten lässt.

7. Verfahren nach einem der Ansprüche 1 bis 6,
**dadurch gekennzeichnet,**
**dass** für den Benutzer eine Modifikationsmöglichkeit an den vorgeschlagenen oder angezeigten geeigneten Trainingsprogrammen vorgesehen ist.

8. Verfahren nach einem der Ansprüche 1 bis 7,
**dadurch gekennzeichnet,**
**dass** bei der Auswahl der ein oder mehreren geeigneten Trainingsprogramme durch die Datenverarbeitungsstation (10) automatisch eine Übereinstimmung weiterer Patientendaten des Patienten mit Patientendaten der Vergleichs-Patienten geprüft und berücksichtigt wird, die zusätzlich in der ersten Datenbank (11) enthalten sind.

9. Verfahren nach Anspruch 8,
**dadurch gekennzeichnet,**
**dass** die weiteren Patientendaten des Patienten eingegeben oder durch die Datenverarbeitungsstation (10) von einer elektronischen Patientenakte abgerufen werden.

10. Verfahren nach einem der Ansprüche 1 bis 9,
**dadurch gekennzeichnet,**
**dass** wiederholt im Verlauf einer Therapie ein aktuelles Fähigkeitsprofil des Patienten bereitgestellt wird, von der Datenverarbeitungsstation (10) automatisch das aktuelle Fähigkeitsprofil des Patienten mit den Vergleichs-Fähigkeitsprofilen verglichen und ein oder mehrere weitere Trainingsprogramme ausgewählt und gegebenenfalls zusammen mit der zugeordneten, gegebenenfalls gemittelten Maßzahl für den Behandlungserfolg angezeigt werden, bei denen das zugeordnete Vergleichs-Fähigkeitsprofil mit dem aktuellen Fähigkeitsprofil des Patienten in einem vorgebbaren Ähnlichkeitsmaß übereinstimmt, um ein dem Patienten bereits verordnetes Trainingsprogramm gegebenenfalls durch ein weiteres Trainingsprogramm zu ersetzen oder zu modifizieren.

11. Verfahren nach Anspruch 10,
**dadurch gekennzeichnet,**
**dass** eine dritte Datenbank (13) bereitgestellt wird, die Überführungsmaßnahmen zwischen unterschiedlichen Trainingsprogrammen enthält, und bei einem Ersatz des verordneten Trainingsprogramms durch ein weiteres Trainingsprogramm von der Datenverarbeitungsstation (10) automatisch Überführungsmaßnahmen zu diesem weiteren Trainingsprogramm durch Rückgriff auf die dritte Datenbank (13) abgerufen und ausgegeben werden.

12. Verfahren nach einem der Ansprüche 1 bis 11,
**dadurch gekennzeichnet,**
**dass** die erste Datenbank (11) mit anonymisierten Daten bereitgestellt wird.

13. System zur Unterstützung bei der Auswahl eines Trainingsprogramms im Rahmen einer Therapieplanung mit einer Datenverarbeitungsstation (10), die mit einer ersten Datenbank (11) verbunden ist, die mehrere zurückliegende Vergleichs-Fähigkeitsprofile von Vergleichspatienten mit einer Zuordnung von jeweils auf Basis dieser Vergleichs-Fähigkeitsprofile verordneten Trainingsprogrammen enthält, und ein Modul (14) zum automatischen Vergleich eines Fähigkeitsprofils des Patienten mit den Vergleichs-Fähigkeitsprofilen und zur Auswahl und Anzeige eines oder mehrerer geeigneter Trainingsprogramme aus der ersten Datenbank (10), bei denen das zugeordnete Vergleichs-Fähigkeitsprofil mit dem Fähigkeitsprofil des Patienten in einem vorgebbaren Ähnlichkeitsmaß übereinstimmt.

14. System nach Anspruch 13,
**dadurch gekennzeichnet,**
**dass** die Datenverarbeitungsstation (10) mit einer zweiten Datenbank (12) verbunden ist, aus denen das Fähigkeitsprofil des Patienten abrufbar ist.

15. System nach Anspruch 13 oder 14,
**dadurch gekennzeichnet,**
**dass** den verordneten Trainingsprogrammen in der ersten Datenbank (11) zusätzlich Maßzahlen für den Behandlungserfolg beim jeweiligen Vergleichspatienten zugeordnet sind und das Modul (14) zur Anzeige der ein oder mehreren geeigneten Trainingsprogramme zusammen mit der zugeordneten, gegebenenfalls gemittelten Maßzahl für den Behandlungserfolg ausgebildet ist.

16. System nach Anspruch 15,
**dadurch gekennzeichnet,**
**dass** das Modul (14) zur Darstellung der geeigneten Trainingsprogramme in der Reihenfolge der Größe der zugehörigen Maßzahlen für den Behandlungserfolg ausgebildet ist.

17. System nach einem der Ansprüche 13 bis 15,
**dadurch gekennzeichnet,**
**dass** das Modul (14) zur Darstellung der geeigneten Trainingsprogramme in der Reihenfolge des Ähnlichkeitsmaßes der Übereinstimmung der zugeordneten Vergleichs-Fähigkeitsprofile mit dem Fähigkeitsprofil des Patienten ausgebildet ist.

18. System nach einem der Ansprüche 15 bis 17,
**dadurch gekennzeichnet,**
**dass** das Modul (14) zur Anzeige nur des oder der geeigneten Trainingsprogramme mit der höchsten Maßzahl für den Behandlungserfolg oder mit einer über einem vorgebbaren Schwellwert liegenden Maßzahl für den Behandlungserfolg ausgebildet ist.

19. System nach einem der Ansprüche 15 bis 18,
**dadurch gekennzeichnet,**
**dass** das Modul (14) für die Ausgabe eines Vorschlags eines geeigneten Trainingsprogramms auf Basis der Übereinstimmung der Vergleichs-Fähigkeitsprofile mit dem Fähigkeitsprofil des Patienten und der Maßzahlen für den Behandlungserfolg der jeweils verordneten Trainingsprogramme nach vorgebbaren Auswahlregeln ausgebildet ist.

20. System nach einem der Ansprüche 13 bis 19,
**dadurch gekennzeichnet,**
**dass** das Modul (14) für den Benutzer eine Modifikationsmöglichkeit an den vorgeschlagenen oder angezeigten geeigneten-Trainingsprogrammen vorsieht.

21. System nach einem der Ansprüche 13 bis 20,
**dadurch gekennzeichnet,**
**dass** das Modul (14) zur automatischen Prüfung und Berücksichtigung einer Übereinstimmung weiterer Patientendaten des Patienten mit Patientendaten der Vergleichs-Patienten ausgebildet ist, die zusätzlich in der ersten Datenbank (11) enthalten sind.

22. System nach Anspruch 21,
**dadurch gekennzeichnet,**
**dass** die Datenverarbeitungsstation (10) Zugriff auf eine elektronische Patientenakte des Patienten hat, aus der die weiteren Patientendaten des Patienten durch das Modul (14) abrufbar sind.

23. System nach einem der Ansprüche 13 bis 22,
**dadurch gekennzeichnet,**
**dass** das Modul (14) für einen wiederholten Abruf des Fähigkeitsprofils des Patienten im Verlauf einer Therapie, den automatischen Vergleich mit den Vergleichs-Fähigkeitsprofilen der ersten Datenbank (11) und die Auswahl und Anzeige eines oder mehrerer geeigneter Trainingsprogramme aus der ersten Datenbank (11) ausgebildet ist, um bei einer Abweichung zu einem bisher verordneten Trainingsprogramm gegebenenfalls ein anderes oder modifiziertes Trainingsprogramm vorzuschlagen.

24. System nach Anspruch 23,
**dadurch gekennzeichnet,**
**dass** die Datenverarbeitungsstation (10) mit einer dritten Datenbank (13) verbunden ist, die Überführungsmaßnahmen zwischen unterschiedlichen Trainingsprogrammen enthält, und das Modul (14) bei einem Vorschlag eines anderen Trainingsprogramms zur Ausgabe von Überführungsmaßnahmen zu diesem anderen Trainingsprogramm durch Rückgriff auf die dritte Datenbank (13) ausgebildet ist.

25. System nach einem der Ansprüche 13 bis 24,
**dadurch gekennzeichnet,**
**dass** die erste Datenbank (11) anonymisierte Daten enthält.
